(19) Europäisches Patentamt
European Patent Office
Office européen des brevets

(11) Veröffentlichungsnummer: **0 025 140**
**B1**

(12) # EUROPÄISCHE PATENTSCHRIFT

(45) Veröffentlichungstag der Patentschrift: **26.01.83**

(51) Int. Cl.³: **C 07 H 15/12**// C07H9/04, C07D211/46

(21) Anmeldenummer: **80104896.8**

(22) Anmeldetag: **16.08.80**

(54) **Verfahren zur Herstellung bekannter und neuer 6-Amino-6-desoxy-2,3-0-isopropyliden-alpha-L-sorbofuranose-Derivate sowie neue Zwischenprodukte des Verfahrens.**

(30) Priorität: **07.09.79 DE 2936240**

(43) Veröffentlichungstag der Anmeldung: **18.03.81 Patentblatt 81/11**

(45) Bekanntmachung des Hinweises auf die Patenterteilung: **26.01.83 Patentblatt 83/04**

(84) Benannte Vertragsstaaten: **AT BE CH DE FR GB IT LI NL SE**

(56) Entgegenhaltungen:
**EP A 0 000 947**
**EP A 0 007 040**

**CHEMISCHE BERICHTE, Band 100, Nr. 3, 1967, Seiten 802-815 Verlag Chemie GmbH Weinheim, DE. H. PAULSEN et al.: « Synthese und Reaktionen von Ketopiperidinosen ».**

(73) Patentinhaber: **BAYER AG**
**Zentralbereich Patente, Marken und Lizenzen**
**D-5090 Leverkusen 1, Bayerwerk (DE)**

(72) Erfinder: **Koebernick, Wolfgang, Dr.**
**Claudiusweg 3**
**D-5600 Wuppertal-1 (DE)**
Erfinder: **Furtwängler, Hans-Rolf, Dr.**
**Tersteegenweg 1**
**D-5600 Wuppertal-1 (DE)**

**EP 0 025 140 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung bekannter und neuer 6-Amino-6-desoxy-2,3-O-isopropyliden-α-L-sorbofuranose-Derivate sowie neue Zwischenprodukte des Verfahrens

Die vorliegende Erfindung betrifft ein chemisch eigenartiges Verfahren zur Herstellung von 6-Amino-6-desoxy-2,3,O-isopropyliden-α-L-sorbofuranose-Derivaten.

Es ist bekannt, daß man die 6-Amino-6-desoxy-2,3-O-isopropyliden-α-L-sorbofuranose erhält, wenn man die 6-Azido-6-desoxy-2,3,O-isopropyliden-α-L-sorbofuranose mit Wasserstoff in Gegenwart von Raney-Nickel hydriert (H. Paulsen, J. Sangster und K. Heyns, Chem. Ber. *100* (1967) 802-815). Nachteilig an diesem Verfahren ist, daß die als Ausgangsmaterial verwendete 2,3,O-Isopropyliden-6-O-tosyl-α-L-sorbofuranose sehr zersetzlich ist und nicht in größeren Mengen gehandhabt werden kann und daß die 6-Azido-6-desoxy-2,3,O-isopropyliden-α-L-sorbofuranose wegen der Azidogruppe zu spontaner exothermer Zersetzung neigt und faher für eine großtechnische Durchführung des Verfahrens ungeeignet ist. Ein weiterer Nachteil des Verfahrens ist die hohe Anzahl von Reaktionsstufen und die damit verbundene geringe Ausbeute.

Weiterhin ist bekannt, daß man 6-Amino-6-desoxy-2,3-O-isopropyliden-α-L-sorbofuranose durch 10-tägiges Erhitzen von 2,3,O-Isopropyliden-6-O-tosyl-α-L-sorbofuranose in flüssigem Ammoniak erhalten kann (K. Tokujama, M. Kiyokawa und N. Hoki, Bull. Chem. Soc. Japan *36* (1963) 1 392-1 395). Das gewünschte Produkt kann nach diesem Verfahren jedoch nicht direkt isoliert werden sondern muß erst mit p-Nitrobenzoylchlorid in die entsprechende N-Benzoylverbindung überführt werden, die isoliert, gereinigt und anschließend zum gewünschten Produkt gespalten wird.

Es wurde nun gefunden, daß man die 6-Amino-6-desoxy-2,3-O-isopropyliden-α-L-sorbofuranose-Derivate der Formel (I)

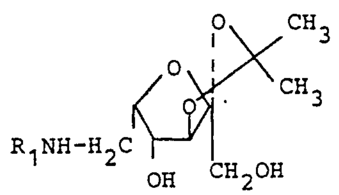

(I)

erhält, worin

$R_1$ Wasserstoff, gegebenenfalls substituiertes Alkyl, Alkenyl, Alkinyl, oder Alkadienyl bedeutet, wenn man die Verbindungen der Formel (II)

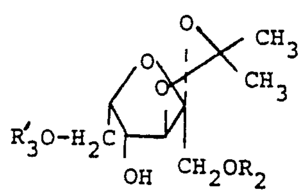

(II)

worin

$R_2$ Wasserstoff oder Acyl und

$R'_3$ den Rest $R_3—SO_2—$ einer Sulfonsäure $R_3SO_3H$, worin $R_3$ ein aliphatischer oder aromatischer Rest ist, bedeuten, im Autoklaven in wäßrigem Medium mit Aminen der Formel (III)

$$R_1—NH_2 \qquad (III)$$

worin

$R_1$ die obengenannte Bedeutung hat, erhitzt, man ein Reaktionsprodukt der Formel

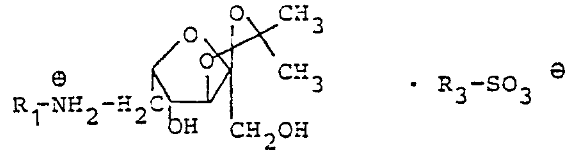

worin

$R_1$ und $R_3$ die obenangegebene Bedeutung haben, in kristalliner Form isoliert, und aus dem Reaktionsprodukt im basischen Medium das freie Amin erzeugt, beispielsweise durch Umsetzen mit Basen oder basischen Ionenaustauschern bei Raumtemperatur bis 100 °C in Wasser während 10 Minuten bis 2 Stunden.

Vorzugsweise bedeutet $R_1$ einen gegebenenfalls substituierten Alkylrest mit 1 bis 30, insbesondere 1 bis 18 C-Atomen, einen Alkenyl-, Alkinyl- oder Alkadienylrest mit 2 bis 18, insbesondere 2 bis 10, C-Atomen.

Als Substituenten für Alkyl seien beispielhaft aufgeführt: Hydroxy, Alkoxy mit 1 bis 4 C-Atomen, Acyloxy, wobei der Acylrest von aliphatischen Carbonsäuren mit 1 bis 7 C-Atomen, aromatischen Carbonsäuren, insbesondere Phenylcarbonsäuren, die im Phenylrest durch Hydroxy, Halogen, $C_1$- bis $C_4$-Alkyl, $C_1$- bis $C_4$-Alkoxy, Nitro und Amino substituiert sein können, heterocyclischen Carbonsäuren, die sich von 5- oder 6-gliedrigen Heterocyclen ableiten, die 1 bis 3 Heteroatome aus der Reihe N, O und S enthalten und im heterocyclischen Ring durch $C_1$- bis $C_4$-Alkyl, Chlor, Brom und Amino substituiert sein können, abgeleitet ist; Amino, Mono- und Dialkylamino mit vorzugsweise 1 bis 4 Kohlenstoffatomen je Alkylrest, Monoacylamino, wobei Acyl die vorstehend genannte Bedeutung hat, Mercapto, $C_1$- bis $C_4$-Alkylthio, Halogen, $C_1$- bis $C_4$-Alkylcarbonyl, Carboxy, Nitro, Cyan, Formyl, Sulfo, Heterocyclyl der vorstehend genannten Definition, $C_3$- bis $C_7$-Cycloalkyl und gegebenenfalls durch Halogen, $C_1$- bis $C_4$-Alkyl, $C_1$- bis $C_4$-Alkoxy, Nitro, Cyano oder Carboxy substituiertes Phenyl.

Halogen ist insbesondere Fluor, Chlor und Brom. Beispiele für Heterocyclen in den vorstehend genannten Definitionen sind Phthalimido, Pyrdinyl, Thienyl, Furyl, Isoxazolyl, Thiazolyl, Glucopyranosyl, Ribofuranosyl und Oxiranyl.

Vorzugsweise sind die Alkylreste $R_1$ unsubstituiert oder durch Hydroxy, $C_1$- bis $C_4$-Alkoxy, Mercapto, $C_1$- bis $C_4$-Alkylthio, Halogen, Nitro, Amino, $C_1$- bis $C_4$-Alkylamino oder $C_1$- bis $C_6$-Alkylcarbonylamino substituiert, wobei der Substituent vorzugsweise nicht an dem den Ringstoff benachbarten Kohlenstoffatom steht.

Acylreste $R_2$ sind insbesondere $C_1$- bis $C_4$-Alkylcarbonylreste.

Sulfonsäurereste $R'_3$ sind insbesondere $C_1$- bis $C_4$-Alkylsulfonyl und gegebenenfalls durch Methyl, Chlor oder Nitro substituiertes Phenylsulfonyl.

Geeignete Basen sind z.B. Alkali- und Erdalkalihydroxide, -carbonate und -alkoholate wie KOH, NaOH, $Na_2CO_3$ und $NaOCH_3$. Als basische Ionenaustauscher können alle handelsüblichen Produkte eingesetzt werden, z.B. Lewatit M 500, M 505, M 600, MP 500, MP 64 oder Dowex IR 400.

Das wäßrige Medium der Umsetzung besteht entweder aus Wasser oder Wasserlösungsmittelgemischen, wobei als Lösungsmittel Ether und Alkohole, vorzugsweise Ethanol und Isopropanol, in Frage kommen. Als Hilfsmittel können quartäre Ammoniumsalze wie ammoniumchlorid oder Tetrabutylammoniumchlorid verwendet werden. Die Umsetzung wird bei Temperaturen zwischen 50 und 250 °C durchgeführt, wobei sich im Autoklaven die den Temperaturen entsprechenden Dampfdrucke der Lösungsmittel einstellen. Die Reaktion kann sowohl im Chargenbetrieb als auch kontinuierlich durchgeführt werden.

Bei den vor der Umsetzung mit Basen oder basischen Ionenaustauschern erhaltenen Zwischenprodukten handelt es sich um die sulfonsauren Salze der erfindungsgemäß herzustellenden Verbindungen mit den Sulfonsäuren $R_3—SO_3H$.

Diese sulfonsauren Salze werden in sehr guter Ausbeute und hoher Reinheit erhalten. Dieses Ergebnis war nach dem Stand der Technik nicht zu erwarten.

Das beschriebene Verfahren weist eine Reihe von Vorteilen auf. So konnte die Anzahl der Reaktionsstufen im Vergleich zu den bekannten Verfahren erheblich reduziert werden. Instabile und gefährliche Vor- und Zwischenstufen werden nicht eingesetzt. Die sulfonsauren Salze gestatten eine einfache Isolierung der erfindungsgemäß herzustellenden Verbindungen aus dem Reaktionsgemisch.

Die Verbindungen der Formel (II) werden in einfacher Weise aus Diaceton-L-sorbofuranose gewonnen, wobei im ersten Schritt die Acylgruppe $R_2$ beispielsweise durch Umsetzung mit Essigsäureanhydrid eingeführt wird und sich im zweiten Schritt nach Abspaltung der Schutzgruppe in 4,6-Position die Sulfonierung im basischen Medium, beispielsweise mit Tosylchlorid oder Mesylchlorid anschließt. Die Aceton-L-sorbofuranose der Formel (IV)

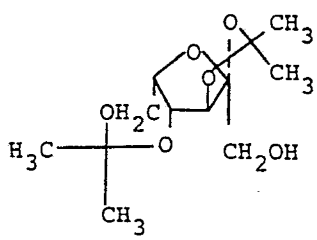

(IV)

ist ein Zwischenprodukt der großtechnisch durchgeführten Vitamin C-Synthese und steht daher in beliebigen Mengen kostengünstig zur Verfügung.

Die Erfindung betrifft weiterhin die als Zwischenprodukte des erfindungsgemäßen Verfahrens durchlaufenden sulfonsauren Salze der Formel (V)

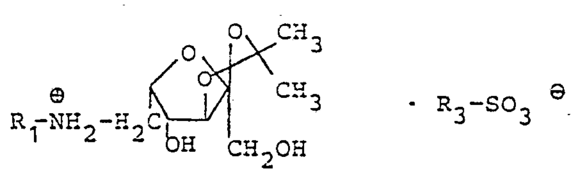

(V)

worin

$R_1$ und $R_3$ die vorstehend angegebene Bedeutung haben.

$R_3$ bedeutet insbesondere p-Tolyl oder Methyl.

Aus den Verbindungen der Formel (I) lassen sich durch Behandeln mit Säure und anschließendes Hydrieren Verbindungen der Formel (VII)

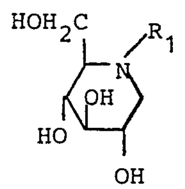

(VII)

worin

$R_1$ die vorstehend genannte Bedeutung hat, gewinnen. Die Verbindungen der Formel (VII) sind potente Inhibitoren für α-Glukosidasen, insbesondere für Disaccharidasen (DE-OS 27 58 025). Sie eignen sich daher als Arzneimittel gegen Diabetes, Hyperlipoproteinämie und Adipositas.

Die Verbindung der Formel (VII) mit $R_1$ ist = H ist in der Literatur als 1-Desoxynojirimycin bekannt.

Insgesamt betrifft daher die Erfindung neue wertvolle Zwischenprodukte sowie ein chemisch eigenartiges Verfahren zur Herstellung von 6-Amino-6-desoxy-2,3-O-isopropyliden-alpha-L-sorbofuranose-Derivaten.

Die Umsetzung der Verbindungen der Formel (I) zu den Verbindungen der Formel (VII) wird beispielsweise durch mehrstündiges Rühren mit zwei N-Salzsäure und anschliessende mehrstündige Hydrierung in Gegenwart von Raney-Nickel bei 3,5 bar unter Zugabe von Triethylamin durchgeführt.

Aus der EP-A 947 ist ein Verfahren zur Herstellung von Verbindungen der Formel (I) bekannt.

Der Vorteil des erfindungsgemäßen Verfahrens gegenüber dem Verfahren der EP-A 947 besteht darin, daß nicht der Aminozucker direkt isoliert wird, sondern daß er in Form eines ausgezeichnet kristallisierenden Ammoniumsalzes einer Sulfonsäure isoliert wird. Es sind dies die Verbindungen gemäß Anspruch 2.

Aufgrund der günstigen Eigenschaften dieser Salze (sehr hohe Reinheit, sehr gut umkristallisierbar) läßt sich der beschriebene Aminozucker in hoher Ausbeute und hervorragender Reinheit isolieren. Alle vorher beschriebenen Verfahren zur Herstellung dieses Aminozuckers sind durch komplizierte mehrstufige Reinigungsschritte in ihrer technischen Brauchbarkeit eingeschränkt.

Geht man bei der Herstellung des Aminozuckers so vor, wie in der EP-A 947 beschrieben, so läßt sich die nach dem erfindungsgemäßen Verfahren erhältliche Ausbeute und Reinheit des Produktes nicht erreichen.

## Beispiel 1

250 g 1-O-Acetyl-2,3-O-isopropyliden-6-O-tosyl-α-L-sorbofuranose (H. Paulsen et al., loc. cit.) werden in einem 5 l Autoklaven mit 1,5 l Ethanol und 150 ml Wasser versetzt. Anschließend werden 750 ml flüssiger Ammoniak zugegeben. Man erhitzt den verschlossenen Autoklaven 3 Stunden auf 140 °C, laßt danach abkühlen, entspannt auf Normaldruck und engt den Autoklaveninhalt im Vakuum bei 70 °C Badtemperatur zum Sirup ein. Danach versetzt man mit 500 ml Isopropanol, kühlt unter Rühren auf 0 °C ab und saugt das ausgefallene Produkt ab. Es wird mit 200 ml Isopropanol nachgewaschen. Man erhält 190,1 g (81 % der Theorie) der Verbindung der Formel

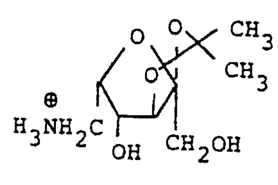

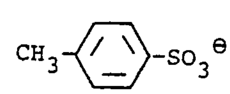

mit dem Schmelzpunkt 202 °C.

200 g des vorstehend beschriebenen Salzes werden bei 50 °C in 800 ml Wasser gelöst, mit 1 000 ml basischem Ionenaustauscher (Lewatit MP 500) versetzt und 30 Minuten bei 50 °C gerührt. Anschließend wird der Ionenaustauscher abfiltriert und gründlich mit Wasser nachgewaschen. Das wäßrige Filtrat wird im Vakuum eingedampft. Man erhält 101 g (90 % der Theorie) der freien Base mit dem Schmelzpunkt 141 °C.

## Beispiel 2

100 g 2,3-O-Isopropyliden-6-O-tosyl-α-L-sorbofuranose werden mit 1 l Ethanol, 80 ml Wasser und 400 mg Ethanolamin versetzt und 4 Stunden im Autoklaven auf 140 °C erhitzt. Danach wird der Reaktionsansatz bei 100 °C und 0,5 Torr eingeengt, mit 1 l Wasser aufgenommen und mit 20 g Aktivkohle

geklärt. Das schwachgelbe waßrige Filtrat wird 30 Minuten mit 700 ml stark basischem Ionenaustauscher (Lewatit MP 500) gerührt, der Ionenaustauscher abfiltriert und das Filtrat im Vakuum zu Sirup eingedampft. Man erhält 40 g (58 % der Theorie) der Verbindung der Formel

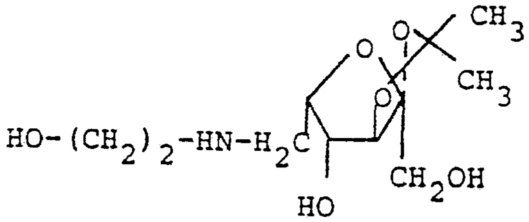

Beispiel 3

100 g 6-Amino-6-desoxy-2,3-O-isopropyliden-α-L-sorbofuranose werden in 40 ml Wasser gelöst und unter Kühlung bei 5 °C langsam mit 84 ml konzentrierter Salzsäure versetzt. Man erwärmt auf 20 °C rührt 2,5 Stunden impft mit einigen Kristallen 6-Amino-6-desoxy-L-sorbose-Hydrochlorid an und tropft dann innerhalb von 2 Stunden 700 ml Ethanol zu der Suspension. Die Suspension wird auf – 5 °C abgekühlt, 1 Stunde bei dieser Temperatur gerührt und dann abfiltriert. Das Kristallisat wird in 700 ml Wasser gelöst, mit 10 g 5 %iger Platin-Kohle versetzt und fünf Stunden im Autoklaven bei Raumtemperatur und 20 Bar $H_2$-Druck hydriert. Die Lösung wird filtriert, am Rotationsverdampfer zum Sirup eingeengt und bei 50 °C mit 300 ml Methanol verrieben. Das Kristallisat wird abgesaugt und getrocknet. Man erhält 58,8 g = 64 % der Theorie 1-Desoxynojirimycin-Hydrochlorid. Schmelzpunkt : 208-210 °C.

**Ansprüche**

1. Verfahren zur Herstellung von 6-Amino-6-desoxy-2,3-O-isopropyliden-α-L-sorbofuranose-Derivaten der Formel

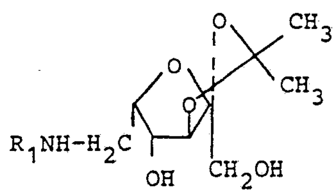

(I)

worin

$R_1$ Wasserstoff, gegebenenfalls substituiertes Alkyl, Alkenyl, Alkinyl, oder Alkadienyl bedeutet, dadurch gekennzeichnet, daß man Verbindungen der Formel

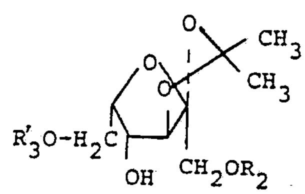

(II)

worin

$R_2$ Wasserstoff oder Acyl und

$R'_3$ den Rest $R_3$—$SO_2$ einer Sulfonsäure $R_3SO_3H$, worin $R_3$ ein aliphatischer oder aromatischer Rest ist, bedeuten, im Autoklaven in wäßrigem Medium mit Aminen der Formel

$$R_1\text{—}NH_2 \quad (III)$$

worin

$R_1$ die obengenannte Bedeutung hat, erhitzt, man ein Reaktionsprodukt der Formel

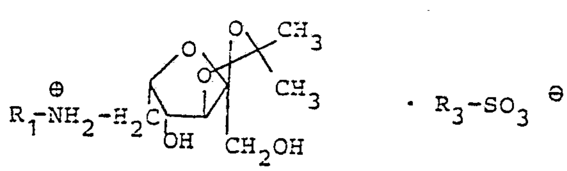

(V)

worin

$R_1$ und $R_3$ die obenangegebene Bedeutung haben, in kristalliner Form isoliert, und aus dem Reaktionsprodukt im basischen Medium das freie Amin erzeugt.

2. Zwischenprodukte der erfindungsgemäßen Verfahrens der Formel

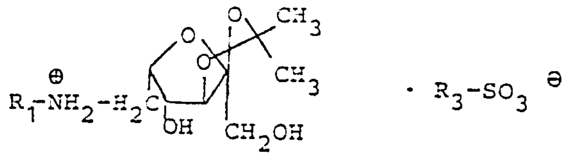

(V)

worin

$R_1$ und $R_3$ die in Anspruch 1 angegebene Bedeutung haben.

**Claims**

1. Process for the production of 6-amino-6-desoxy-2,3,-O-isopropylidene-α-L-sorbofuranose derivatives of the formula

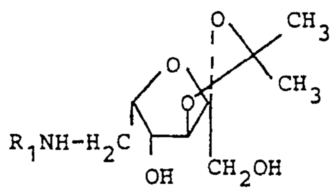

(I)

wherein

$R_1$ denotes hydrogen, optionally substituted alkyl, alkenyl, alkinyl or alkadienyl, characterised in that compounds of the formula

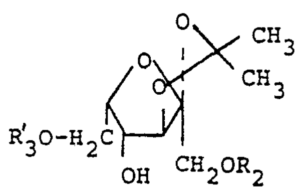

(II)

wherein

$R_2$ denotes hydrogen or acyl and

$R'_3$ denotes the radical $R_3—SO_2—$ of a sulphonic acid $R_3SO_3H$, wherein $R_3$ is an aliphatic or aromatic radical, are heated in autoclaves in an aqueous medium with amines of the formula

$$R_1—NH_2 \qquad (III)$$

wherein

$R_1$ has the abovementioned meaning, a reaction product of the formula

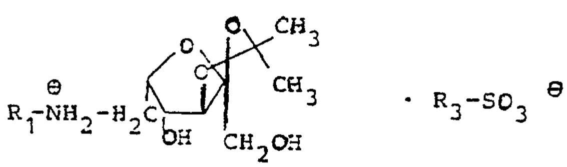

(V)

wherein

$R_1$ and $R_3$ have the abovementioned meaning, is isolated in crystalline form and the free amine is produced from the reaction product in a basic medium.

2. Intermediate products of the process according to the invention of the formula

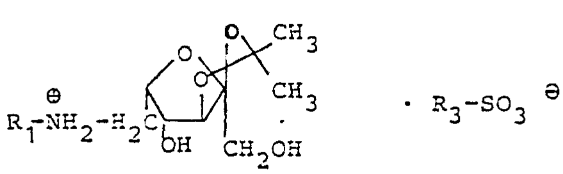

(V)

wherein

$R_1$ and $R_3$ have the meaning indicated in Claim 1.

**Revendications**

1. Procédé de préparation de dérivés du 6-amino-6-désoxy-2,3-O-isopropylidène-alpha-L-sorbofurannose de formule

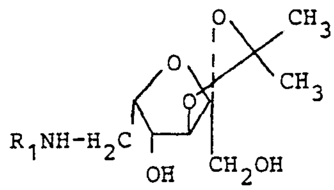

(I)

dans laquelle

$R_1$ représente l'hydrogène, un groupe alkyle, alcényle, alcynyle ou alcadiényle éventuellement substitué, caractérisé en ce que l'on chauffe des composés de formule

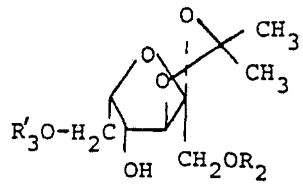

(II)

dans laquelle

$R_2$ représente l'hydrogène ou un groupe acyle, et

$R'_3$ représente le reste $R_3$—$SO_2$— d'un acide sulfonique $R_3SO_3H$, $R_3$ représentant un reste aliphatique ou aromatique, à l'autoclave en milieu aqueux avec des amines de formule

$$R_1—NH_2 \qquad (III)$$

dans laquelle

$R_1$ a la signification indiquée ci-dessus, on isole à l'état cristallisé un produit de réaction de formule

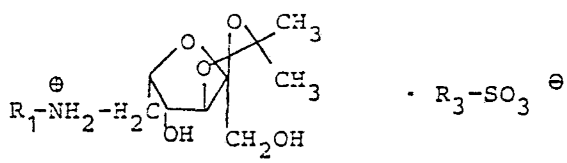

(V)

dans laquelle

$R_1$ et $R_3$ ont les significations indiquées ci-dessus, et à partir du produit de réaction, on forme l'amine libre en milieu basique.

2. Produits intermédiaires du procédé selon l'invention, de formule

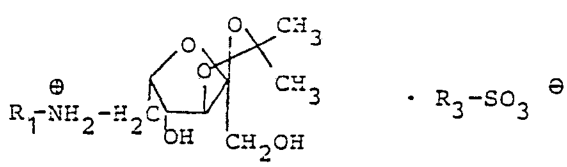

(V)

dans laquelle

$R_1$ et $R_3$ ont les significations indiquées dans la revendication 1.